# EUROPEAN PATENT APPLICATION

(11) **EP 2 875 820 A1**
(43) Date of publication of application: **27.05.2015**
(21) Application number: 12874726.8
(22) Date of filing: 21.08.2012
(51) Int. Cl.: A61K 35/64, A61K 31/592, A61K 31/593, A61P 19/10

(54) **PREPARATION AND METHOD FOR THE PROPHYLAXIS AND TREATMENT OF OSTEOPOROSIS AND BONE FRACTURES**

(30) Priority: 19.04.2012 RU 2012115653
(71) Applicant: Obshestvo S Ogranichennoj Otvetstvennostju "PARAFARM", Penza 440026 (RU)
(72) Inventor: STRUKOV, Villorij Ivanovich, Penza Penzenskaya obl. 440011 (RU); JHONES, Olga, Fort Wors, TX 76107 (US); KRUTIAKOV, Evgenij Nikolaevich, Penza 440062 (RU); ELISTRATOV, Konstantin Gennad'evich, Penza Penzenskaya obl. 440061 (RU)
(74) Representative: Wablat Lange Karthaus
(86) International application number: PCT/RU2012/000689
(87) International publication number: WO 2013/157984

(57) **Abstract**

The method for preventing and treating osteoporosis and bone fractures and the preparation for preventing and treating osteoporosis and bone fractures relates to medicine, in particular to agents for the treatment and prevention of conditions associated with various forms of osteoporosis. The method is characterised in that it involves taking between 10 mg and 1000 mg per day of drone brood, between 50 IU and 1,000,000 IU per day of vitamin D or vitamins of this group and/or the active metabolites thereof, and between 25 mg and 3000 mg per day of a calcium compound, so as to be simultaneously supplied to the body daily, and the preparation composed of between 10 mg and 1000 mg of drone brood, between 50 IU and 1,000,000 IU of vitamin D or vitamins of this group and/or the active metabolites thereof and between 25 mg and 3000 mg of a calcium compound, can be in powder, tablet or capsule form. Implementing the invention provides for the achievement of the technical result stated by the author, which makes it possible to eliminate or reduce the mineralisation imbalance of various bone tissue segments. The use of the claimed agent strengthens the mechanism for uniformly restoring bone mineral density, the use of drone brood in combination with vitamin D aims to provide for additional strength in remodelling injured bone tissue segments and for bone tissue retention by maintaining androgen levels.

## Description

The invention relates to medicine, in particular to agents for the treatment and prevention of conditions associated with various forms of osteoporosis.

The prior art discloses that, in connection with the discovery of D vitamins and the subsequent hormonal forms of vitamin D, the focus for treating opsteopenia, osteoporosis and osteomalacia shifted to this group of preparations (cholecalciferol, ergocalciferol, videhol, vigantol, One-Alpha, catcitriol, etc.). Calcium and vitamin D preparations were combined to enhance calcium absorption. Medications such as 'Kaltsii D3 Nikomed', 'Kaltsimen Advans', 'Kaltsimin', 'Tsitrokal', 'Alfadol kaltsiya' etc. are now widely used. The disadvantage of these agents is that: calcium and vitamin D preparations intensify the activity of one another, and therefore there is a great risk of over mineralising various tissues and organs to the point of calcinosis, i.e. irreversible changes in the body of the patient. Uncontrolled use of calcium preparations may result in a drug-induced pathology, the calcification of small and large vessels, the formation of kidney stones may as well as stones in other organs.

The prior art discloses that maintaining normal bone mineral density in both young and old people plays an important role for masculine and feminine sex hormones. Low sex hormone levels in the body are one of the leading causes of osteoporosis. People have tried to treat osteoporosis by exogenous replacement therapy of these hormones. However, the treatment was unsuccessful: high doses of testosterone cause a pharmacological disaster and female hormones increase the risk of oncopathology.

Osteoporosis is a metabolic skeletal disease, which is characterised by a reduction in bone mass per unit volume and by the microarchitectural deterioration of bone tissue, which lead to a reduction in the amount of calcium in the bones and to a high fracture risk for any bone including the hip.

The prior art closest to the invention proposed in this text is the biologically active food additive to prevent diseases caused by osteoporosis (the preparation 'Osteomed' which is composed of calcium and drone brood in various ratios) (patent No. 2412616, A23L1/30, 2009).

The following disadvantages where found when testing 'Osteomed' on 36 patients with postmenopausal osteoporosis over 12 months:
1. A long course of treatment is required in order to achieve a positive effect.
2. The cavities grew smaller in 75% of patients, while the cavities in 25% of patients did not change.

The below agent is proposed in order to eliminate these disadvantages:
- between 50 IU (international units) and 1,000,000 IU of vitamin D or vitamins of this group (and/or the active metabolites thereof) per day;
- between 10 mg and 1000 mg of drone brood per day; and
- between 25 mg and 3000 mg of one of the acceptable calcium compounds per day at the following ratio of ingredients:
   The claimed agent can be in tablet, powder or capsule form and divided into one or more doses per day.

The technical result consists in eliminating or reducing the mineralisation imbalance of various bone tissue sections. Using the claimed agent enhances the mechanism for uniformly restoring bone mineral density, and using drone brood in combination with vitamin D additionally aims to enhance the remodeling of injured bone tissue sections and to retain bone tissue by supporting androgen levels.

This result is achieved by the method for preventing and treating osteoporosis and bone fractures, which involves taking between 10 mg and 1000 mg of drone brood per day, between 50 IU and 1,000,000 IU of vitamin D or vitamins of the this group and/or the active metabolites thereof per day, and between 25 mg and 3000 mg of a calcium compound per day; and by the preparation for preventing and treating osteoporosis and bone fractures, which is composed of drone brood, vitamin D or vitamins of that group and/or the active metabolites thereof and calcium compounds; and according to the invention drone brood, vitamin D or vitamins of this group and/or the active metabolites thereof, and the calcium compound are supplied to the body at the same time over the course of a day in powder, tablet or capsule form.

The method for preventing and treating osteoporosis and bone fractures is carried out in the following way.

The studies carried out by the authors have shown that the combined use of vitamin D, calcium and drone brood makes it possible to increase the speed with which cavities are closed and correspondingly to increase the effectiveness of osteoporosis treatment by 40%, which can be confirmed by a number of illustrative examples using statistical methods.

The claimed preparation for preventing and treating osteoporosis and bone fractures was used in tablet form for the study in the following composition (one tablet containing):
500 IU of vitamin D3,
100 mg of drone brood, and
0.5g of calcium citrate.

Example 1. The author carried out a study on the comparative effectiveness of 'osteomed' and the preparation presented by the author in 56 female patients with postmenopausal osteoporosis. Depending on the treatment method, 56 women aged between 47 and 72 years of age with postmenopausal (primary) osteoporosis were divided into two groups with comparable ages and disease severity:
Group 1 (27 women) received 5 tablets of Osteomed per day (2 in the morning and 3 at night).
Group 2 (29 women) received 5 tablets of the claimed preparation (2 in the morning and 3 at night).

Both preparations were given over 3 months followed by a month break. The bone mineral density of all of the patients and the change in the dimensions of the cavities was measured using a radiological absorptive method on a DTX-100 apparatus (Denmark) before being prescribed the agents and after every three month period of therapy until the end of treatment. Only this type of apparatus allows for the simultaneous measurement of bone mineral density, cavities in bones; and excess salt deposits in soft tissue and the changes therein. The results of the studies on the mineral density and dimension changes of the cavities in the groups after 6 months of therapy are seen below in table 1.

**Table 1. The comparative effectiveness of treatment for postmenopausal osteoporosis depending on the selected preparation after 6 months of therapy.**

| Group | n-number of people | Change in cavity (M±m%) | | |
|---|---|---|---|---|
| | | Positive absolute no./% | Without changes absolute no./% | Negative absolute no./% |
| 1 (Osteomed) | 27/100% | 12/44±10% including closed 27±5% | 9/33±9 | 6/22±8 |
| 2 (claimed preparation) | 29/100% | 20/69±9% including closed 9/ 31±9% | 6/21 ±8 | 3/10±6 |

The table shows that the speed with which cavities were closed with the claimed preparation was quicker than that of osteomed.

### Example 2. The following observation served as an example of the effectiveness of claimed agent for treating osteoporosis in comparison with the preparation Kaltsii D3 Nikomed.

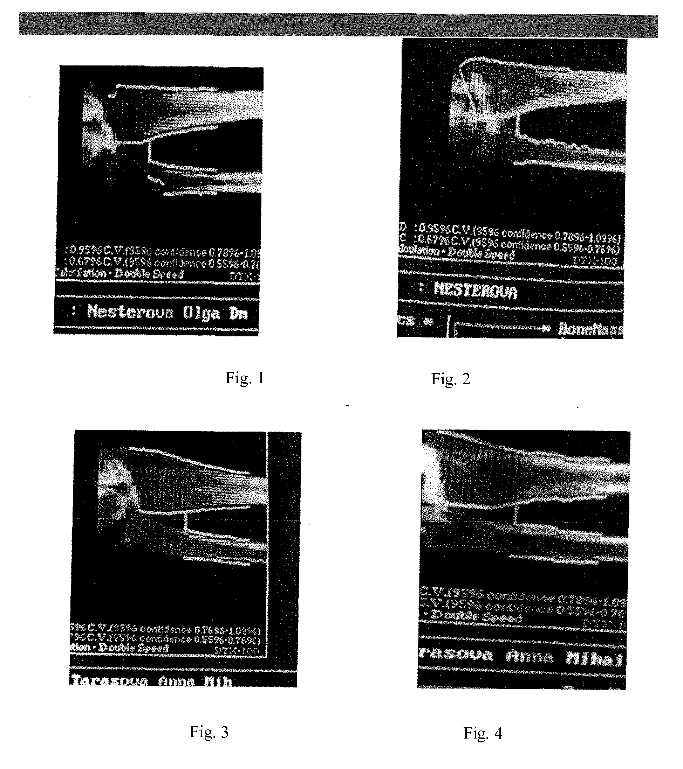

A 59-year-old woman, N.O.D., received Kaltsii D3 Nikomed for osteoporosis over a year without any positive changes. Osteometry revealed cavities (Figure 1 prior to treatment) for which this woman was prescribed the claimed preparation, and after 10 months of treatment the cavities were closed (see Figure 2, records of study number 1667 and number 1977).
Figure 1. Cavities prior to the commencement of treatment using the claimed agent.
Figure 2. There are no cavities after 10 months of treatment and there is an improvement in bone structure and mineralisation.

### Example 3. Closing the cavities using the claimed agent for treating osteoporosis.

72-year-old female patient, T.A.M, 165 cm tall, weighing 74 kg, and went through the menopause 21 years previous. Over the course of the year this patient received Kaltsii D3 Nikomed without any positive changes. The record of osteometric examination No. 1189 prior to treatment: T-score ^ - 3.9 CO; - 3.7; - 4.1 CO. Cavities in trabecular bone sections. Diagnosis: primary osteoporosis with a risk of falling and bone fractures. Figure 3 shows an osteodensitogram prior to treatment. Figure 4 shows an osteodensitogram after completing a course of treatment with the claimed preparation (record of examination No. 1668).

The scope of the range of the claimed preparation is determined by the characteristics of the patient: their age, eating habits, lifestyle, race, country of habitation, sex and genetic and previous diseases. A doctor assessing these criteria selects the specific proportion of the constituent parts of the claimed agent and adjusts this based on the change in the closing of the cavities. The weight of the patient affects the dosing of the preparation. Slender women with white skin are more prone to osteoporosis than dark skinned or heavier women. The fatty tissue in heavier women produces oestrogen which prevents the formation of osteoporosis. Heavier women are rarely diagnosed with osteoporosis. Therefore, the heavier the woman, the smaller the dose.

An explanation of the range limits:
1) Between 50 IU and 1,000,000 IU per day of vitamin D or vitamins from this group (and/or the active metabolites thereof). The lower end is the effective dose while the upper end is a toxic dose.
2) Between 10 IU to 1000 IU per day of drone brood. The lower end is the effective dose while the upper end is the feasibility of use in terms of the ratio of effectiveness/price rise of the product.
3) Between 25 mg and 3000 mg per day of a calcium compound. The lower end is the effective dose while the upper end is a toxic dose.

Although the components of the claimed preparation are known in folk and traditional medicine, the combination thereof in one product is not known, specifically, the discovered synergistic effect makes it possible to solve the problem of balanced bone mineralisation in both trabecular bone sections and cortical bone sections and to solve the problem of interest to achieve the claimed technical result, namely to eliminate or reduce an imbalance in the mineralisation of various bone tissue sections.

Vitamin D is introduced because drone brood is saturated with vitamins particularly vitamin D3, but in non-replaceable, small doses. Therefore, the concentration thereof is insufficient for treating osteoporosis.

Drone brood has to be introduced as a donor of the following sex hormones: estradiol, progesterone and testosterone, which have a positive effect on bone mineralisation. Drone brood serves as a substrate for synthesising its own sex hormones (estradiol and testosterone) which stimulate the reproductive functions in men and women. Brood, which is saturated with the hormones of beneficial insects (male bee), stimulates the central mechanisms for regulating the reproductive organs and eliminates the need for replacement therapy. Only the combination of the stated preparations makes it possible to improve calcium absorption and to deposit same in bone sections with low mineralisation, which are also known as cavities. This is confirmed by the results of radiological absorptive osteometry. The use of the claimed agent enhances the mechanism for supplementing calcium in the body because using calcium compounds with vitamin D aims to additionally supply calcium, and the simultaneous use of drone brood facilitates the retention thereof by maintaining androgen levels. The combined use of vitamin D with calcium and drone brood makes it possible to achieve the greatest effectiveness in osteoporosis therapy, to reduce the frequency of adverse side effects in the form of calcified deposits and kidney stones.

The examples below illustrate the effectiveness of the claimed agent in comparison with the prior art.

### Example no. 1. Female patient F, 64 years old, postmenopausal osteoporosis had significant cavities when examined:

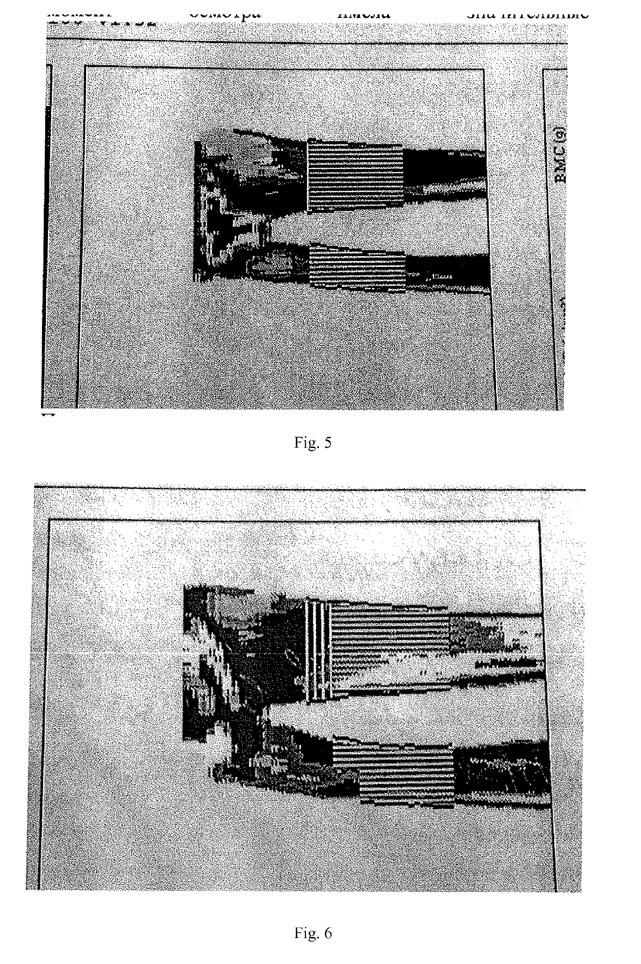

The cavities in the picture are orange (see Fig. 5).

After 6 months of treatment with the claimed agent, the cavities were significantly reduced (see Fig. 6).

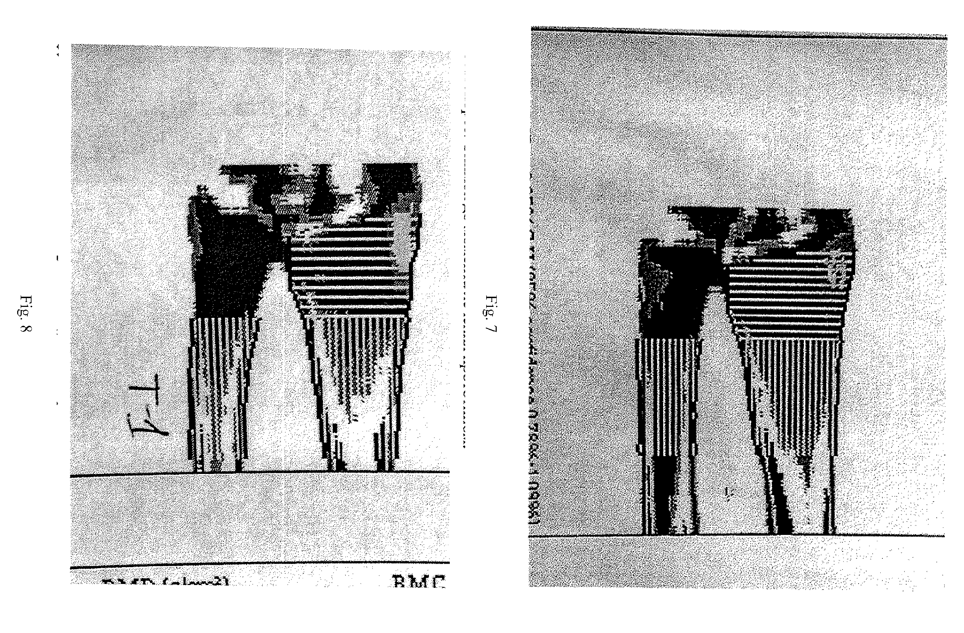

After 9 months of treatment, the cavities were even further reduced (see Fig. 7).

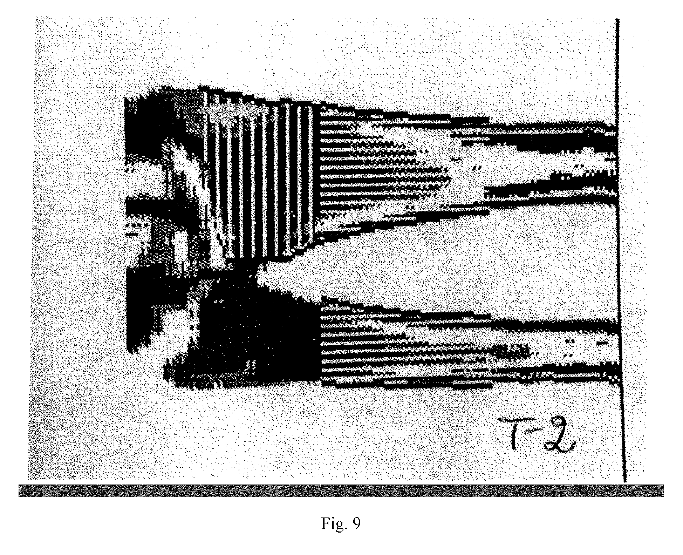

Treatment with the prior art had a lesser effect on the patients over a longer period of time, which indicates that the claimed agent is superior to the prior art. This can be seen in the example for treating female patient T., 62 years old with postmenopausal osteoporosis. Fig. 8 is a picture before treatment with the prior art. The reduction of cavities in patient T after 9 months of treatment with the prior art is shown in Fig. 9.

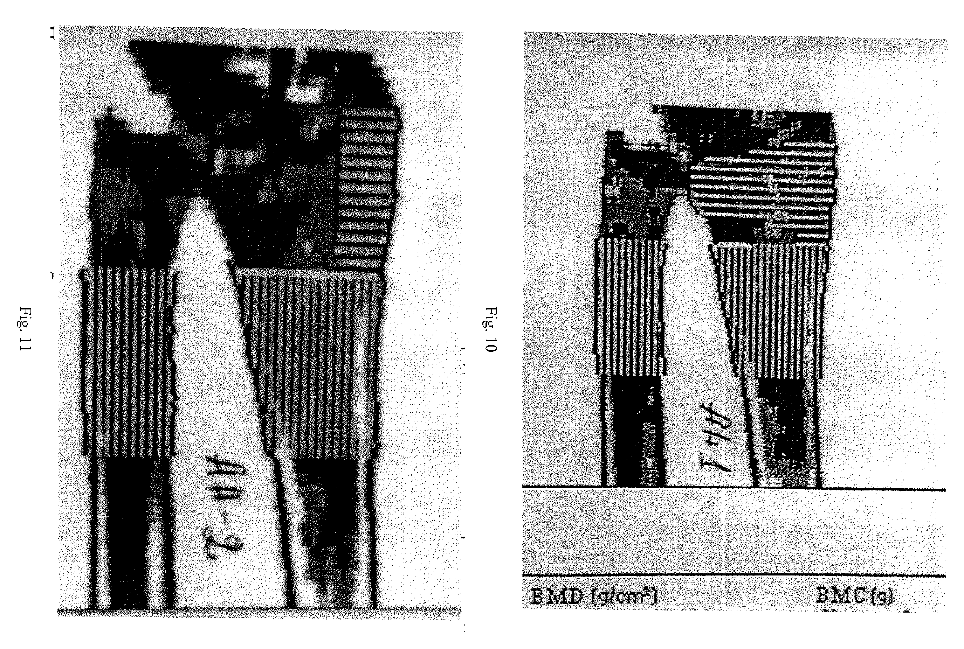

### Example No.2. Patient AA, 70 years of age, postmenopausal osteoporosis. The patient had significant cavities prior to treatment. (see Fig. 10).

The cavities were closed after 9 months of treatment with the claimed agent. (see Fig. 11)

A significantly worse change in cavity closures was observed in the treatment with the prior art:

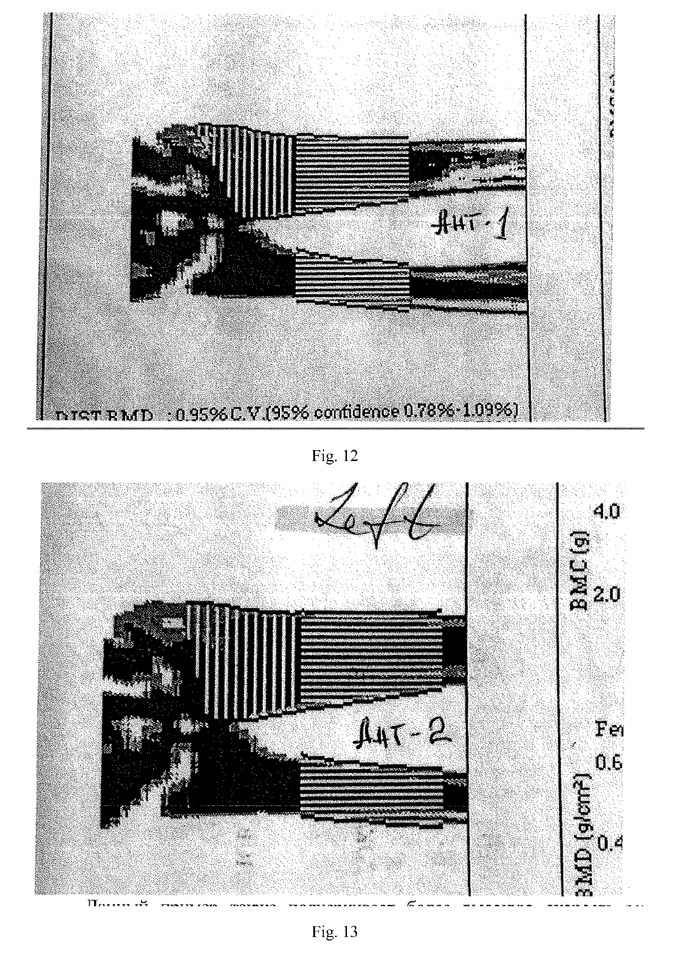

Female patient A.N.T., 70 years of age, postmenopausal osteoporosis, has cavities (see Fig. 12).

After 9 months of treatment with the prior art, the cavities were reduced but were not closed (see Fig. 13).

This example also highlights the greater speed with which the cavities are closed with the claimed agent in comparison to the prior art, and means it is more effective. Therefore, a doctor can influence the speed with which the cavities are closed by prescribing the claimed agent.

It should be noted that the higher the dose of the claimed agent, the quicker the initial speed with which the cavities are closed, after which the speed is reduced, irrespective of the dose. When prescribing large doses, the doctor must always take into account the danger of hypercalcemia and vitamin D toxicity, and must only prescribe these strictly based on the evidence of the case.

It is considered appropriate to use a dose of vitamin D (or the active metabolites thereof) for a number of diseases, in a dose of 1,000,000 IU once per month and for further treatment to be carried out using an agent composed of a calcium and drone brood compound.

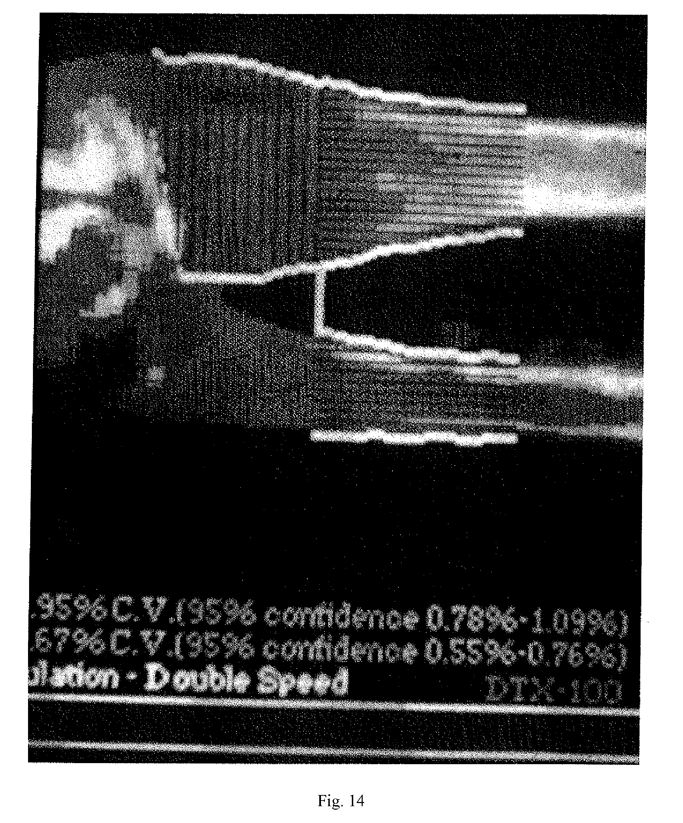

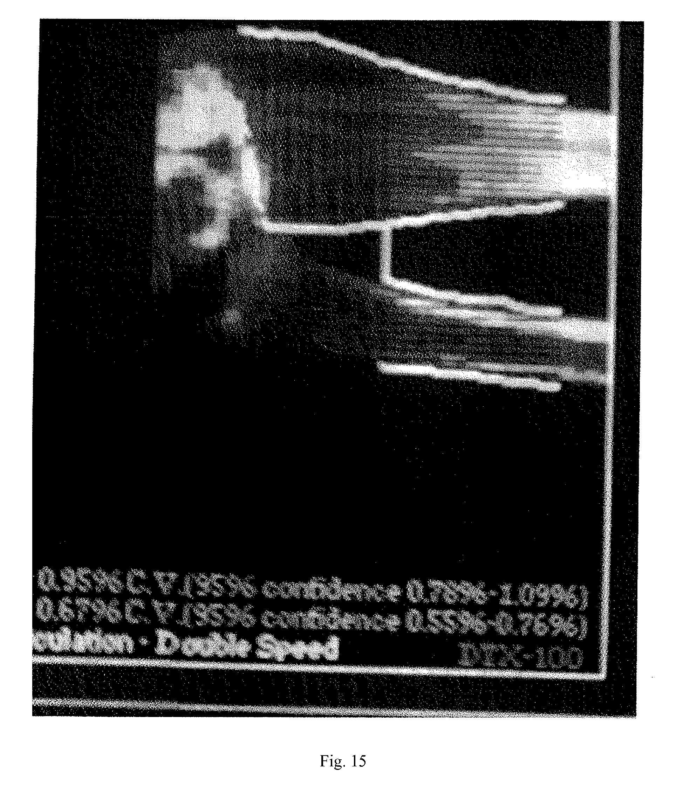

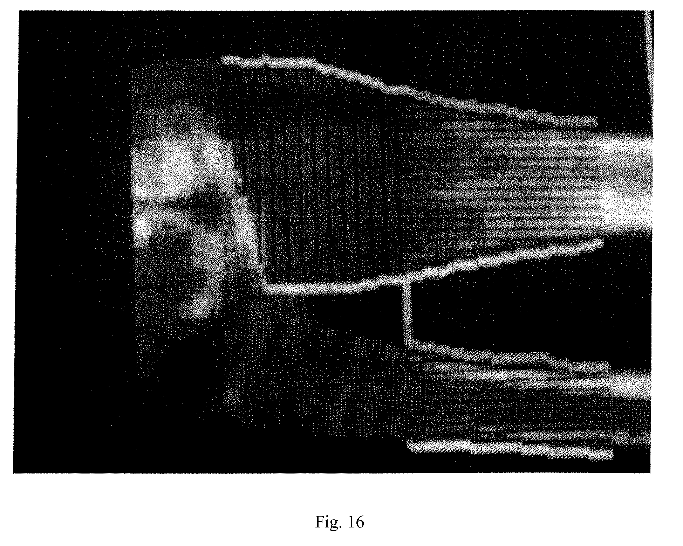

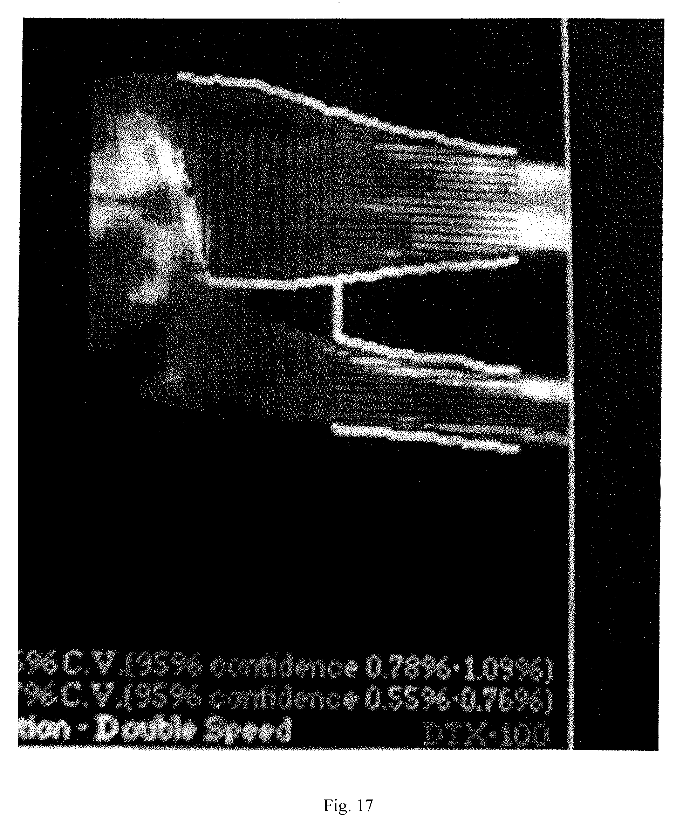

An example of the impact large doses of the claimed agent have on speed with which cavities are closed:
Female patient T., 72 years old. Took the claimed agent having a composition of:
   500 IU of vitamin D3 + 100 mg of drone brood + 0.5 g calcium citrate in 5 tablets (2 in the morning and 3 at night). The speed with which the cavities closed was shown after every two months of the study (see Figures 14-17).

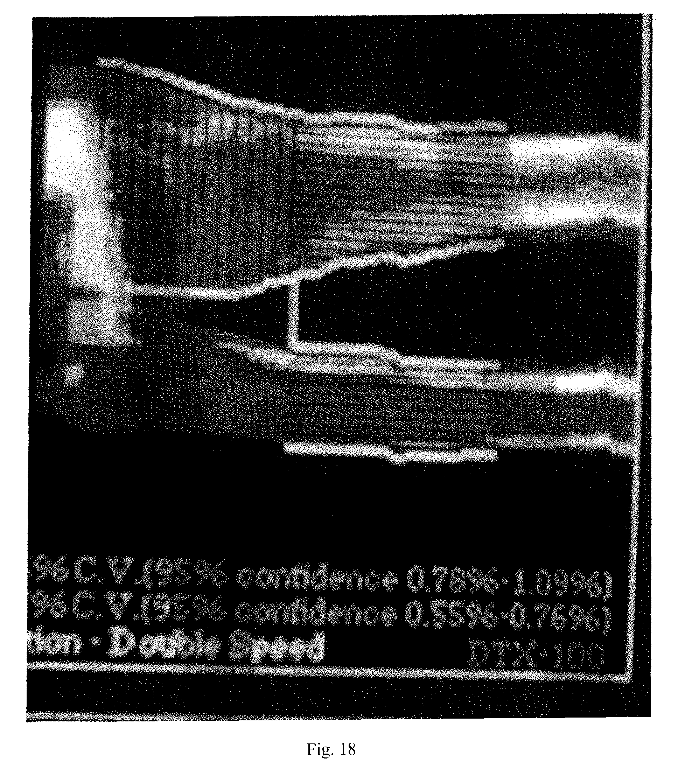

Cavity closures after 10 months are shown in Fig 18.

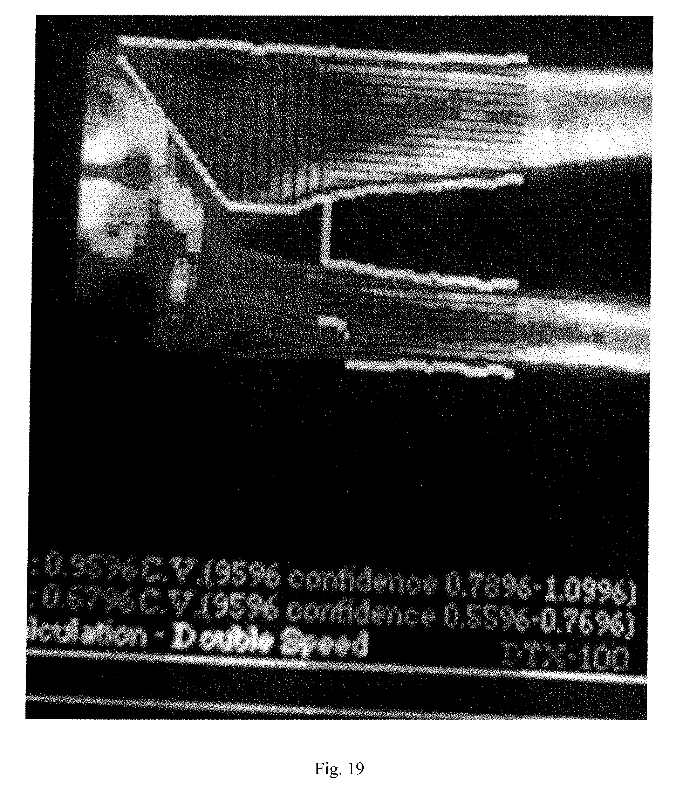

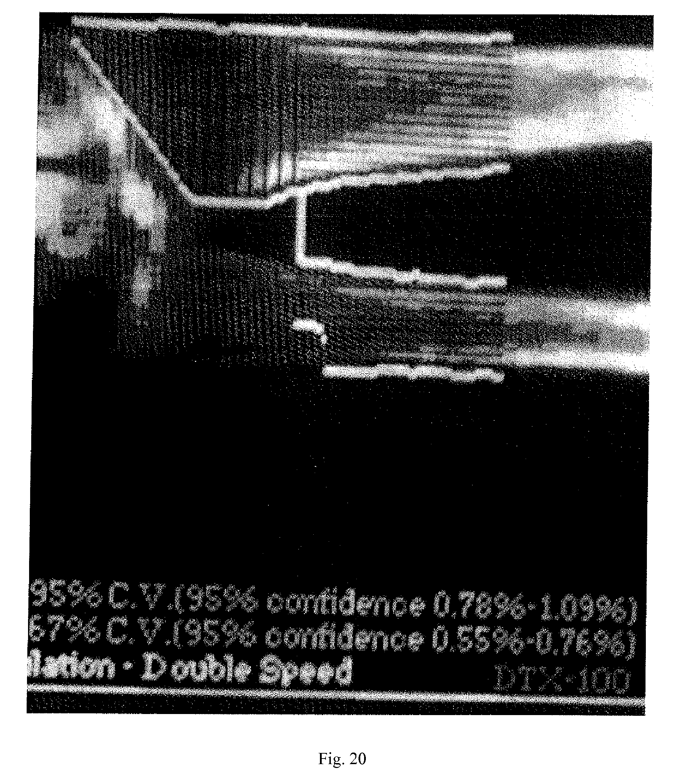

Female patient B., 72 years old. This patient took the claimed agent in powder form in a daily dose having the composition: 50,000 IU of vitamin D3 + 1000 mg of drone brood + 3g of calcium citrate. The speed with which the cavities closed was shown after every 2 months of the study (see Figures 19-20).

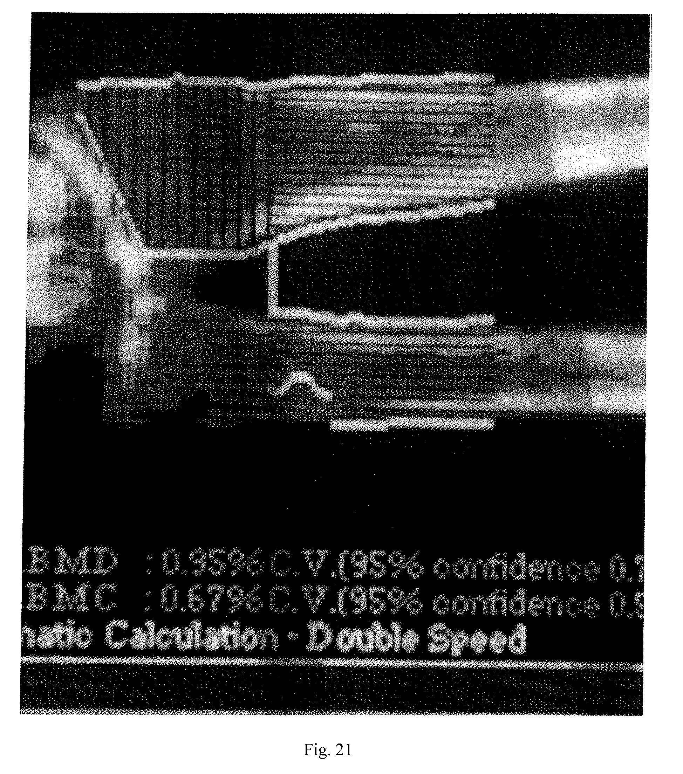

In other words, after 6 months the cavities were closed. (see Fig. 21)

Women taking the claimed agent in the period after menopause should do so constantly with short breaks (no longer than 1 month). Extended breaks cause the cavities to reform.

When prescribing the claimed agent, the doctor chooses the dosage of the constituent parts thereof individually based on the condition of the disease of the patient and the negative effect that vitamin D and large doses of calcium has on the body as a whole. The condition of the patient should be assessed using density measuring apparatuses by which the cavities are measured every 6-9 months and the composition of the claimed agent is adjusted.

## Claims

1. A method for preventing and treating osteoporosis and bone fractures, which involves taking between 10 mg and 1000 mg per day of drone brood, between 50 IU and 1,000,000 IU per day of vitamin D or vitamins of this group and/or the active metabolites thereof, and between 25 mg and 3000 mg per day of a calcium compound.

2. The method according to claim 1, which is **characterised in that** the drone brood, vitamin D or vitamins of this group and/or the active metabolites thereof and the calcium compound are simultaneously supplied to the body daily.

3. The preparation for preventing and treating osteoporosis and bone fractures, which is composed of between 10 mg and 1000 mg of drone brood, between 50 IU and 1,000,000 IU of vitamin D or vitamins of this group and/or the active metabolites thereof and between 25 mg and 3000 mg of a calcium compound.

4. The preparation according to claim 2, which is **characterised in that** it is provided in powder, tablet or capsule form.
